(19) Europäisches Patentamt
European Patent Office
Office européen des brevets

(11) **EP 3 206 016 A1**

(12) **EUROPEAN PATENT APPLICATION**
published in accordance with Art. 153(4) EPC

(43) Date of publication:
**16.08.2017 Bulletin 2017/33**

(21) Application number: **15848344.6**

(22) Date of filing: **07.10.2015**

(51) Int Cl.:
*G01N 21/359* (2014.01)    *G01N 29/024* (2006.01)

(86) International application number:
**PCT/JP2015/078472**

(87) International publication number:
**WO 2016/056590 (14.04.2016 Gazette 2016/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **10.10.2014   JP 2014209123**

(71) Applicant: **Osaka University**
**Suita-shi, Osaka 565-0871 (JP)**

(72) Inventors:
• **ITOZAKI, Hideo**
  **Suita-shi**
  **Osaka 565-0871 (JP)**
• **AKABA, Hideo**
  **Suita-shi**
  **Osaka 565-0871 (JP)**

(74) Representative: **J A Kemp**
**14 South Square**
**Gray's Inn**
**London WC1R 5JJ (GB)**

(54) **LIQUID EXAMINATION DEVICE AND LIQUID EXAMINATION METHOD**

(57) Provided is an examination technique that can quickly and reliably examine, from the outside of a container, whether a liquid which fills the container contains an explosive or the like, without being influenced by such things as the light transmissivity or size of the container, or the amount of liquid remaining in the container or the position of a label. In the present invention, a liquid examination device is formed by integrating the following: a near infrared-light examination device that examines whether a liquid which fills a container contains an explosive, an explosive raw material, and/or an illicit drug by projecting near infrared light into the liquid from outside of an optically transparent container, receiving the near infrared light which passed through the liquid or the near infrared light which was scattered by the liquid, and analyzing the absorption spectrum of such light; and an ultrasonic wave examination device that examines whether a liquid which fills a container contains an explosive, an explosive raw material, and/or an illicit drug by receiving the reflected waves of ultrasonic waves projected towards the liquid from outside of a metal container, and analyzing the ultrasonic wave speed of such waves.

[FIG.1]

## Description

[TECHNICAL FIELD]

The present invention relates to a liquid examination device for examining a liquid filled in a container. More particularly, the present invention relates to a liquid examination device for examining whether an explosive, a raw material for explosive, or an illicit drug of some kind is contained in a liquid and to a liquid examination method conducted by using said liquid examination device.

[BACKGROUND ART]

Including the simultaneous terrorist bomb explosion incidents that occurred in Great Britain in 2005, terrorist bomb explosion incidents have occurred frequently at public facilities and public transportation in recent years. In recent times, there have been growing cases where a terrorist, pretending to be a passenger carries onto an airplane or the like an optically transparent container for a drink, such as a PET bottle or a glass bottle, or an aluminum beverage container, for example, filled with a liquid in which an explosive, a raw material for explosive, or the like has been mixed or dissolved. Further, there have also been growing cases where a liquid, in which an illicit drug such as a narcotic or a stimulant has been dissolved, is smuggled in a manner that a container is filled with the illicit drug.

As measures against the carrying of, a raw material for explosive, and illicit drugs (hereafter, collectively say as "an explosive or the like") on airplanes, hand luggage examinations are conducted on passengers at airports for preventing occurrence of incidents such as terrorist incidents, and smuggling. In order to conduct examinations on many passengers, there is a need to swiftly conduct the examinations, but it is not easy to determine whether or not a liquid with which a container has been filled contains an explosive or the like during short-time examination.

Under such circumstances, the inventors of the present invention have developed liquid examination method and device for examining whether an explosive or the like is contained in a liquid, by irradiating the liquid filled in an optically transparent container with near-infrared light and based on absorption spectrum of the near-infrared that has transmitted through the liquid or scattered by the liquid.

[PRIOR ART DOCUMENT]

[PATENT DOCUMENT]

Patent Document 1: JP-B-5207462
Patent Document 2: JP-A-2011-220710

[SUMMARY OF THE INVENTION]

[PROBLEMS TO BE SOLVED BY THE INVENTION]

According to the above-described technique, in order to prevent influence of extraneous light on the irradiated near-infrared light, examination is conducted with an object of examination set in a space of a prescribed size to which entrance of extraneous light blocked. Therefore, there has been limits of size and shape of containers to be the objects of measurement and examination. For instance, it has been difficult to apply the technique to a container of a large diameter.

Further, it has been necessary to set a container in a prescribed attitude. Therefore, measurement becomes difficult when only a small amount of liquid is left in the container, or when a label is on an interfering position.

Further, a metal container such as an aluminum can or a steel can does not transmit light and, therefore, the technique could not be applied to examine a liquid filled in such a container.

Therefore, there has been a strong demand for an examination technique that can promptly and reliably examine whether a liquid filled in a container contains any explosive or the like from outside of the container, independent of the size, whether the container is optically transparent or not, the remaining amount of liquid in the container or the position of any label.

[MEANS OF SOLVING THE PROBLEMS]

The present inventors conducted extensive studies in order to solve the above problems. As a result, it was found that the above problems can be solved by inventions of the Claims shown below, and thus the present invention has been completed.

According to claim 1, the present invention provides

a liquid examination device for checking whether an explosive, a raw-material for explosive and/or an illicit drug is contained in a liquid filled in a container, comprising, as integrally formed components:

a near-infrared light examination device irradiating said liquid with near-infrared light from outside an optically transparent container, receiving said near-infrared light transmitted through the liquid or said near-infrared light scattered by the liquid, and analyzing absorption spectrum of the received light, and thereby examining whether an explosive, a raw material for explosive and/or an illicit drag is contained in the liquid filled in said optically transparent container; and
an ultrasonic examination device receiving reflected wave of ultrasonic wave applied to said liquid from outside a metal container, analyzing the ultrasonic velocity and thereby examining whether an explosive, a raw material for explosive and/or an illicit drag is contained in the liquid filled in said metal container.

Conventionally, near-infrared light examination devices and ultrasonic examination devices have been studied as liquid examination devices for examining whether an explosive or the like is contained in a container.

A near-infrared light examination device irradiates a liquid in a container with near-infrared light, and analyzes absorption spectrum of the near-infrared light transmitted through the liquid or of the near-infrared light scattered by the liquid, and thereby examines whether or not any explosive or the like is contained therein. Since it is known that absorption spectra differ liquid by liquid, it is possible to check if any explosive is contained or not by analyzing the absorption spectra.

On the other hand, an ultrasonic examination device applies ultrasonic wave to a liquid in a container through a wall surface of the container, analyzes ultrasonic velocity obtained from reflected wave propagated through the liquid and reflected by an opposite wall surface of the container, and thereby examines whether or not any explosive or the like is contained therein. Here again, since it is known that the ultrasonic velocity differs liquid by liquid, it is possible to check if any explosive or the like is contained or not by analyzing the ultrasonic velocity.

It is noted, however, that since the near-infrared light examination device examines by irradiating a liquid in a container with near-infrared light, if the liquid is filled in a metal container, even when near-infrared light is emitted, the near-infrared light does not reach the liquid and, hence, examination is impossible.

On the other hand, the ultrasonic examination device examines by calculating the ultrasonic velocity from the time necessary for the reflected wave to reach and be reflected back from the container wall surface and from the distance and time of this round trip. Therefore, measurement of distance is of high importance. However, a plastic container such as a PET bottle is prone to deformation, causing errors in measuring the distance of ultrasonic wave propagation. This makes accurate examination difficult.

As described above, conventionally, a near-infrared light examination device or an ultrasonic examination device has been selectively used to check if any explosive or the like is contained or not depending on whether the container is optically transparent or not. It is not easy, however, to install the near-infrared light examination device and the ultrasonic examination device in combination, considering the cost and space for installing these devices. In addition, it takes considerable time to conduct examination.

The invention according to this claim, however, solves these problems by combining and integrating the near-infrared examination device and ultrasonic examination device, and provides a liquid examination device that can examine whether any explosive or the like is contained or not in a speedy and reliable manner from outside of a container, independent of whether the container is optically transparent or not.

The invention according to claim 2 provides the liquid examination device according to claim 1 wherein analysis of absorption spectrum of said near-infrared light by said near-infrared light examination device is conducted by comparing with database formed in advance on relations between explosives, raw materials for explosives and/or illicit drugs and their absorption spectra of near infrared light.

As described above, the absorption spectrum of near-infrared light passed through a liquid or of near-infrared light scattered by a liquid differs liquid by liquid. Therefore, by forming and storing database of these relations in advance and comparing the absorption spectrum of near-infrared light obtained by the near-infrared light examination device with the stored database, it is possible to easily extract the absorption spectrum of an analogous liquid and, thus, to examine promptly whether any explosive or the like is contained.

The invention according to claim 3 provides the liquid examination device according to claim 1 or 2, wherein said near-infrared light examination device includes two poles erected approximately vertically and spaced by a prescribed distance from each other, and a liquid in an optically transparent container placed between said two poles is irradiated with near-infrared light.

In the invention according to this claim, the infrared examination device includes two poles that are erected approximately vertically and spaced by a prescribed distance from each other, and it is configured to irradiate a liquid in an optically transparent container placed between the two poles with near-infrared light.

Therefore, it is possible to irradiate a liquid with near-infrared light and to examine it regardless of the size or shape of a container containing the liquid. Further, it is also possible to set the container at a tilt or to put it upside down as appropriate in accordance with the amount of liquid remaining in the container or in accordance with a label position. Therefore, it is possible to promptly and reliably examine containers of various types as to whether any explosive or the like is contained, regardless of the remaining amount of liquid in the container or the label position.

The invention according to claim 4 provides the liquid examination device according to claim 3 wherein an irradiating means for irradiating a liquid contained in an optically transparent container placed between said two poles with near-infrared light is provided on each of said two poles, and
a light receiving means for receiving near-infrared light transmitted through the liquid or near-infrared light scattered by the liquid is provided on either one of said two poles.

In the invention according to this claim, the near-infrared light examination device includes a near-infrared light irradiating means provided on each pole and a light receiving means for receiving said near-infrared light on one of the poles.

Accordingly, the light receiving means can receive two types of near-infrared light, that is, the near infrared light transmitted through the liquid and the near-infrared light scattered by the liquid.

As a result, it is possible to examine a transparent liquid filled in a transparent container by mainly receiving near-infrared light (transmitted light) that has been emitted from the irradiating means of the opposite pole and transmitted through the liquid (transmitting method).

On the other hand, it is possible to examine a highly scattering liquid such as milk filled in a transparent container or a transparent liquid filled in a highly scattering container referred to as opaque, by mainly receiving near-infrared light that has been emitted from the irradiating means of the same pole and reflected and scattered by the liquid (reflecting method).

As described above, by the invention according to this claim, examination can be conducted with the method of receiving near-infrared light automatically switched between the transmitting method and the reflecting method in accordance with the intensity of scattering of the container or the liquid. Therefore, it is possible to promptly and reliably examine various containers and various liquids as to whether any explosive or the like is contained.

The invention according to claim 5 provides the liquid examination device according to claim 4 wherein said light receiving means includes a light receiving mirror, and said received near-infrared light is reflected by said light receiving mirror before the absorption spectrum of said near-infrared light is measured.

In the invention according to this claim, the light receiving means is provided with a light receiving mirror, said received near-infrared light is reflected by said light receiving mirror, and thereafter, the absorption spectrum of said near-infrared light is measured. Therefore, it becomes possible to house a measuring means for measuring the absorption spectrum at a position away from the poles. Thus, the liquid examination device can be made more compact.

The invention according to claim 6 provides the liquid examination device according to claim 4 or 5, wherein

irradiation of near-infrared light by said irradiating means and reception of near-infrared light by said light receiving means are conducted constantly.

Since irradiation of near-infrared light by the irradiating means and reception of near-infrared light by the light receiving means are conducted constantly, examination can be started immediately after a container is set and, therefore, it is possible to promptly examine whether any explosive or the like is contained.

The invention according to claim 7 provides the liquid examination device according to claim 6 wherein said near-infrared light examination device includes a reference data updating function of updating reference data based on a light source spectrum of said near-infrared light received immediately before.

When examination is to be done with high accuracy using the near-infrared light examination device, it is necessary to remove influence of atmosphere such as extraneous light, by correcting obtained data of absorption spectrum using data of light source spectrum (reference data) obtained when no object of examination is set.

For this purpose, in a conventional near-infrared light examination device, received near-infrared light is split into two, to separately obtain reference data and the spectrum of the object of examination. This leads to a problem that the time of measurement becomes longer.

In the invention according to this claim, as described above, irradiation of near-infrared light by the irradiating means and reception of near-infrared light by the light receiving means are conducted constantly, and the reference data is updated based on the light source spectrum of near-infrared light received immediately before. As a result, it becomes unnecessary to obtain reference data at the time of examination. Therefore, the device structure can be made simpler and examination can be done with higher efficiency.

In the invention according to this claim, the reference data is updated such that if the light source spectrum of received near-infrared light is not much changed and within a prescribed range as compared with the light-source spectrum of near-infrared light received immediately before, the light source spectrum of received near-infrared light is used as the reference data.

The invention according to claim 8 provides the liquid examination device according to claim 6 or 7, wherein placement of said optically transparent container between said two poles is detected by a reduction of said received near-infrared light, and examination by said near-infrared light examination device is automatically started.

In the invention according to this claim, as described above, irradiation of near-infrared light by the irradiating means and reception of near-infrared light by the light receiving means are conducted constantly. Therefore, when an optically transparent container is placed between the two poles, the near-infrared light at the light receiving means reduces, and the light source spectrum of near-infrared light significantly changes as compared with the light source spectrum of the near-infrared light received immediately before.

Since placement of a container can be detected from the significant change in light source spectrum of near-infrared light, examination can automatically be started based on the detection. As a result, speedier examination becomes possible, eliminating the necessity of, for example, pressing an examination start switch.

The invention according to claim 9 provides the liquid examination device according to any one of claims 1 to 8, wherein

the device has, as database, absorption spectra of liquids known in advance as explosives, raw-materials for explosives and/or illicit drugs and of liquids known in advance as safe substances,

correlation coefficient of absorption spectrum of a measured liquid and each of the absorption spectra recorded in said database is calculated, and

by selecting the closest spectrum from said database based on said calculated correlation coefficient, examination is conducted as to whether the measured liquid is an explosive, a raw-material for an explosive and/or an illicit drug, or a safe substance.

In the invention according to this claim, the absorption spectra of liquids known in advance as explosives, raw-materials for explosives and/or illicit drugs and of liquids known in advance as safe substances are provided in the form of database, and based on the correlation coefficient between the absorption spectrum of measured liquid and each of the absorption spectra in the database, the liquid in the container is identified and whether it is a dangerous or safe substance is determined. Thus, accurate examination is possible.

The invention according to claim 10 provides the liquid examination device according to any one of claims 1 to 9, wherein analysis of the ultrasonic velocity by said ultrasonic examination device is conducted by comparing with database formed in advance on relations between explosives, raw materials for explosives and/or illicit drugs and their ultrasonic velocities.

As described above, the ultrasonic velocity obtained from reflected wave of the ultrasonic wave applied to a liquid differs liquid by liquid. Therefore, by forming and storing database of these relations in advance and comparing the ultrasonic velocity obtained by said ultrasonic examination device with the stored database, it is possible to easily extract the ultrasonic velocity of an analogous liquid and, thus, to examine promptly whether any explosive or the like is contained.

The invention according to claim 11 provides the liquid examination device according to claim 10, comprising

an ultrasonic pulse transmitting/receiving device transmitting an ultrasonic pulse to said liquid from outside of a metal container and applying the ultrasonic pulse to said liquid, and receiving reflected wave of said ultrasonic wave,

a propagation path length measuring device measuring propagation path length based on a diameter of said metal container,

a propagation time measuring device measuring propagation time based on time interval of said reflected wave received when said ultrasonic pulse is reflected a number of times, and

a temperature measuring device measuring temperature of said metal container.

Using the ultrasonic pulse transmitting/receiving device, the propagation path length measuring device, the propagation time measuring device and the temperature measuring device, it is possible to easily obtain the ultrasonic velocity in a liquid and, thus, to examine promptly whether any explosive or the like is contained.

The invention according to claim 12 provides the liquid examination device according to claim 11, wherein

said ultrasonic examination device may include a container receiving surface on which said metal container is set, and a pressing member pressing said metal container set on said container receiving surface to a head section transmitting/receiving an ultrasonic signal of said ultrasonic pulse transmitting/receiving device, and

said propagation path length measuring device may calculate the propagation path length in said metal container based on the position of said pressing member.

In the invention according to this claim, a metal container is set on the container receiving surface of the ultrasonic examination device, and using the pressing member, it is pressed against the ultrasonic pulse transmitting/receiving device, specifically to a head section (hereinafter also simply referred to as a "head section") transmitting/receiving an ultrasonic signal of the ultrasonic pulse transmitting/receiving device. Therefore, examination can be done without any space generated between the container and the head section of ultrasonic pulse transmitting/receiving device and between the metal container and the pressing member.

At this time, since it is possible to know the propagation path length utilizing the distance between the head section of the ultrasonic pulse transmitting/receiving device and the pressing member, the propagation path length can easily be calculated from the position of pressing member when the metal container is pressed against the ultrasonic pulse transmitting/receiving device.

Here, it is preferable to use, as a specific propagation path length measuring device, a variable resistor of which electric resistance changes linearly in accordance with length. Such a variable resistor has a simple structure, and by linking the pressing member and the variable resistor, the propagation path length can automatically be measured from the magnitude of electric resistance.

The invention according to claim 13 provides the liquid examination device according to claim 11 or 12, wherein

said ultrasonic examination device may examine whether or not said liquid contains an explosive, a raw-material for an explosive or an illicit drug,

based on said measured propagation path length and the ultrasonic velocity in said liquid calculated from said propagation path length and said propagation time, and on the temperature of said liquid measured separately.

Since the ultrasonic velocity is temperature-dependent, at the time of examination, it is preferable to examine presence of an explosive or the like considering the temperature dependency. Specifically, database based on relations between ultrasonic velocities and liquids is prepared in consideration of the temperature dependency, and examination of an explosive or the like is conducted by comparison therewith. Thus, it is possible to easily conduct a highly accurate examination.

The invention according to claim 14 provides the liquid examination device according to claim 12 or 13, wherein

the examination by said ultrasonic examination device may be automatically started upon detection of said metal container pressed against the head section transmitting/receiving an ultrasonic signal of said ultrasonic pulse transmitting/receiving device.

By detecting that the metal container is pressed against the head section of said ultrasonic pulse transmitting/receiving device, examination can be started automatically. As a result, speedier examination becomes possible, eliminating the necessity of, for example, pressing an examination start switch.

The invention according to claim 15 provides the liquid examination device according to any one of claims 1 to 14, wherein

an alarm means for notifying the result of examination by light and/or sound is provided, further.

An examiner must immediately know the result of examination by the above-described near-infrared examination device or the ultrasonic examination device and to respond quickly. In the invention according to this claim, light and/or sound is used as the alarm means and, therefore, it is possible to know the result of examination promptly and reliably and to regulate any liquid that contains an explosive or the like.

The invention according to claim 16 provides the liquid examination method for examining whether an explosive, a raw-material for explosive and/or an illicit drug is contained in a liquid filled in a container using the liquid examination device according to any one of claims 1 to 15.

By an examination technique using an above described liquid examination device, it can be examined promptly and reliably whether a liquid filled in a container contains any explosive or the like from outside of the container, independent of the size or whether the container is optically transparent or not, regardless of the remaining amount of liquid in the container or the label position.

[EFFECT OF THE INVENTION]

According to the present invention, an examination technique can be provided wherein it can be examined promptly and reliably whether a liquid filled in a container contains any explosive or the like from outside of the container, independent of the size or whether the container is optically transparent or not, regardless of the remaining amount of liquid in the container or the label position.

[BRIEF DESCRIPTION OF THE DRAWINGS]

[FIG. 1] A perspective view of a liquid examination device in accordance with an embodiment of the present invention.

[FIG. 2] Perspective views explaining the results of examination carried out by a liquid examination device in accordance with an embodiment of the present invention.

[FIG. 3] Schematic illustrations showing the pressing member of ultrasonic examination device; (a) shows before setting the metal container, (b) shows after setting the metal container.

[FIG. 4] Perspective views explaining an ultrasonic examination by the ultrasonic examination device in accordance with an embodiment of the present invention.

[FIG. 5] A figure showing deviation between the first echo time T and each of echo intervals $\tau$.

[FIG. 6] A figure showing temperature dependency of sound velocity for various kinds of liquids.

[EMBODIMENTS FOR CARRYING OUT THE INVENTION]

An embodiment of the present invention will be described below with reference to the drawings.

1. Overall structure of the liquid examination device

At first, the overall structure of liquid examination device according to this embodiment is explained.

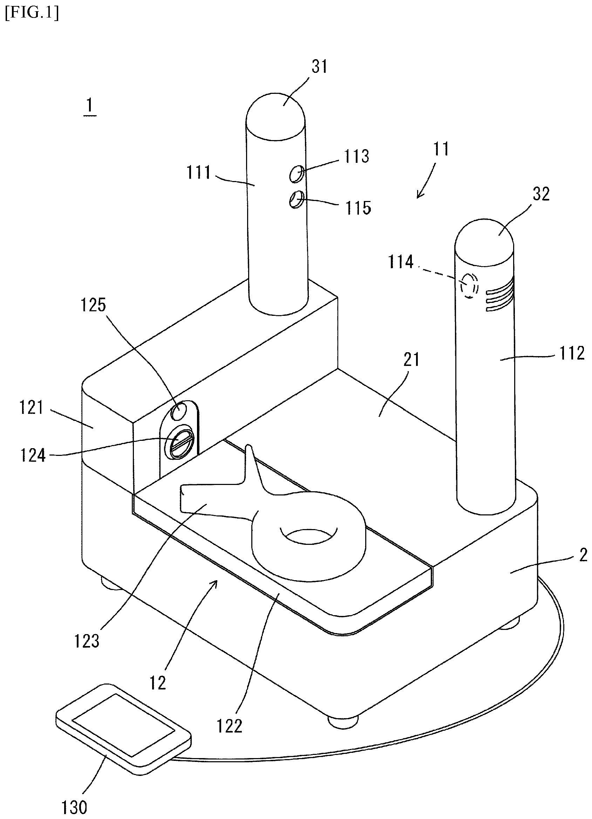

Fig. 1 is a perspective view of the liquid examination device in accordance with an embodiment of the present invention. As shown in Fig. 1, a liquid examination device 1 in accordance with the present embodiment has a near-infrared light examination device 11 and an ultrasonic examination device 12, integrally formed on a body 2 of the examination device.

Near-infrared light examination device 11 includes first pole 111 and second pole 112, spaced by a prescribed distance from each other, erected approximately vertically on one side of body 2 of examination device. The first pole 111 has a first near-infrared light irradiating means 113 provided on the side opposite to the second pole 112, and the second pole 112 has a second near-infrared light irradiating means 114 provided on the side opposite to the first pole 111. (Though the second near-infrared light irradiating means 114 is behind the view and actually invisible, it is depicted in dotted lines here for easier understanding.)

Ultrasonic examination device 12 includes a container receiving section 121 erected approximately vertically on an upper base 21 of body 2 of examination device, with a head section 124 of ultrasonic pulse transmitting/receiving device and a sensor unit 125 of temperature measuring device provided on its container receiving surface. Further, on base 21, a pressing member is provided, which includes a stage 122 mounted movable toward/away from container receiving section 121 and a holder 123 for pressing the container set on stage 122 to the head section 124 of ultrasonic pulse transmitting/receiving device.

At upper end portions of the first pole 111 and/or second pole 112, an LED 31/32 is provided as an indicator for indicating the result of examination, indicating whether or not an explosive or the like is contained in the liquid in the container in different colors, for example, green if the liquid does not contain any explosive or the like and red if the liquid contains an explosive or the like.

Body 2 of examination device and container receiving section 121 include a built-in micro-computer chip or the like, storing procedures and database used at the time of examining an explosive or the like in the liquid by near-infrared light examination device 11 and by ultrasonic examination device 12, as will be described later.

A display unit 130 is a small PC (personal computer) or a tablet terminal connected to the body 2 of examination device, for displaying various contents of control and results of examination by ultrasonic examination device 12.

Near-infrared light examination device 11 and ultrasonic examination device 12 will be described below separately in order.

2. Near-infrared light examination device

(1) Near-infrared light examination device

First, the near-infrared light examination device will be described. A conventional mounted-type near-infrared light examination device typically has a cover, and a near-infrared light irradiating/receiving means is provided inside the cover. After a container is set in the near-infrared light irradiating/receiving means, examination is done with a lid closed, in order to prevent decrease in examination accuracy caused by the influence of extraneous light. For this reason, it takes time and effort to set and examine a container and, in addition, reduction in size of the examination device has been difficult.

In contrast, as can be seen from Fig. 1, according to the present embodiment, there is no cover or lid, the near-infrared light receiving means 115 is provided on a side surface of approximately vertically standing first pole 111, and the near-infrared light emitted from the first near infrared light irradiating means 113 and the second near infrared light irradiating means 114 and transmitted through or scattered by the liquid is received thereby.

Here, if a halogen lamp is used as a light source of near-infrared light emitted from the first near infrared light irradiating means 113 and the second near infrared light irradiating means 114, light of high intensity can be emitted. Further, since the near-infrared light receiving means 115 is provided in a horizontal direction, it is not much susceptible to the influence from above such as room illumination, and thus, the influence of extraneous light can be reduced.

When a transparent container filled with transparent liquid is to be examined, near-infrared light emitted by the second near infrared light irradiating means 114 and transmitted through the liquid is received by near-infrared light receiving means 115 (transmitting method), and when a highly scattering liquid or container is to be examined, near-infrared light emitted by the first near infrared light irradiating means 113 and reflected and scattered by the liquid is received by near-infrared light receiving means 115 (reflecting method), whereby various types of containers and liquids can promptly and reliably be examined as to whether it contains any explosive or the like.

Further, in the present embodiment, a light receiving mirror (not shown) is provided on near-infrared light receiving means 115, and the received near-infrared light is reflected by the receiving mirror and transmitted by an optical fiber to a built-in spectrometer in the body of examination device, where measurement of absorption spectrum of the near-infrared light is conducted. Since the near-infrared light is transmitted to the spectrometer that measures the absorption spectrum, it becomes possible to measure the absorption spectrum at a position away from the poles, enabling more compact device design. Further, since the influence of extraneous light can sufficiently be reduced, examination with higher accuracy becomes possible.

Further, according to the present embodiment, while the liquid examination device 1 is in operation, the first near infrared light irradiating means 113 and the second near infrared light irradiating means 114 are adapted to constantly irradiate near-infrared light and near-infrared light receiving means 115 is adapted to constantly receive the near-infrared light, even when no object is set for examination.

The reference data is updated using the absorption spectrum of near-infrared light received immediately before. As a result, different from the conventional near-infrared light examination devices, it becomes unnecessary to measure the absorption spectrum by dividing the received near-infrared light to reference data and measurement data. Thus, the device structure can be made simpler and examination can be done with higher efficiency.

Further, since the near-infrared light is constantly irradiated and received, it is possible to automatically start examination by detecting setting of a container. Specifically, when a container is placed between the first pole 111 and the second pole 112, the amount of light received by near-infrared light receiving means 115 reduces (light reduction), and the absorption spectrum of near-infrared light significantly changes from the absorption spectrum of near-infrared light received immediately before. Thus, placement of the container can be detected. Based on this detection, examination can be started automatically.

The space between the first pole 111 and the second pole 112 is set appropriately such that various types of containers can be accepted and that setting of a container in a tilted manner is possible if such setting becomes necessary due to restrictions such as the amount of liquid left in the container or the label position.

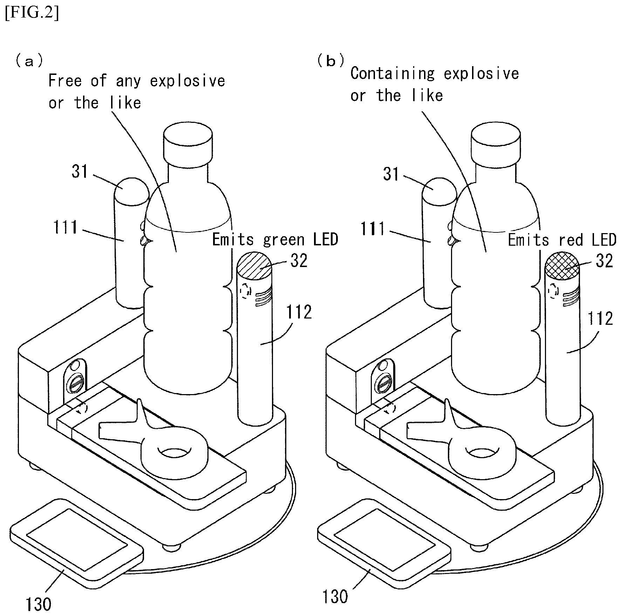

Fig. 2 includes perspective views showing the results of examination carried out by the near-infrared light examination device. When the liquid in a container is free of any explosive or the like, LED 32 emits green light as shown in Fig. 2(a), notifying the examiner that the liquid does not contain any explosive or the like and it is safe. On the other hand, if the liquid in the container is an explosive or the like, LED 32 emits red light as shown in Fig. 2(b), warning the examiner that the liquid possibly contains an explosive or the like.

In this manner, the result of examination is immediately indicated by LED 32. Therefore, it is possible to promptly and reliably know the result of examination and to appropriately regulate any liquid containing an explosive or the like. In addition to LED 32 on the second pole 112, an LED 31 may similarly be mounted on the first pole 111 and indications may be given by both LEDs 31 and 32 simultaneously.

(2) Near-infrared light examination method

Next, the liquid examination method using the near-infrared light examination device above (the near-infrared light examination method) will be described. In this near-infrared examination, by way of example, near-infrared light having the wavelength of 650 nm to 1000 nm is used.

Prior to examination, absorption spectra of liquids known to be dangerous substances such as explosives (for example, hydrogen peroxide solution) and absorption spectra of scattered light of liquids known to be safe substances (for example, commercially available general beverages and household products such as cosmetics) are measured and stored as database in the near-infrared light examination device.

Next, absorption spectrum of near-infrared light of the liquid as the object of examination is measured and compared with various absorption spectra recorded as the database, and correlation coefficients with the absorption spectrum of the object of examination are calculated (correlation analysis). From the results of comparison using correlation analysis, data having the highest similarity to the object of examination is selected and thereby the liquid is identified, and whether it is a dangerous substance or safe substance is determined.

3. Ultrasonic examination device

Next, ultrasonic examination device will be explained. This ultrasonic examination device is used for examining an explosive or the like in a liquid in a metal container.

(1) Ultrasonic examination device

As described above, as shown in Fig. 1, ultrasonic examination device 12 includes a container receiving section 121 erected approximately vertically on an upper base 21 of body 2 of examination device is provided and a head section 124 of ultrasonic pulse transmitting/receiving device and a sensor unit 125 of temperature measuring device provided on the container receiving surface. Further, on base 21, a stage 122 mounted movable toward/away from container receiving section 121 and a pressing member having a holder 123 for pinching and pressing the metal container set on stage 122 to the head section 124 of ultrasonic pulse transmitting/receiving device are provided.

Ultrasonic examination device 12 includes a propagation time measurement device for measuring propagation time based on time interval of reflected waves received when ultrasonic pulse is reflected a number of times, which is provided inside container receiving section 121.

Head section 124 of the ultrasonic pulse transmitting/receiving device is provided on the wall surface of container receiving section 121, and it transmits an ultrasonic pulse to the liquid from outside the set metal container to apply the ultrasonic pulse to the liquid, and receives reflected ultrasonic pulse. For the head section 124 of ultrasonic pulse transmitting/receiving device as such, an ultrasonic sensor, for example, may be used.

A preferable ultrasonic sensor includes an ultrasonic sensor using a piezoelectric element. An ultrasonic sensor using a piezoelectric element is a dual type sensor having an integrated transmitting/receiving transducer, which is preferred because transmitting circuit and receiving circuit can be formed separately and can be controlled easily.

The propagation time measuring device measures the propagation time based on the time interval of reflected waves received when an ultrasonic pulse is reflected a number of times, as mentioned above. Specifically, the head section 124 of ultrasonic pulse transmitting/receiving device transmits an ultrasonic pulse having a prescribed frequency and prescribed interval to the liquid in a container, and when echo signals (reflected waves) are received, propagation time can be calculated based on time difference between the pulse reflected twice or more.

Sensor unit 125 of the temperature measuring device is provided above head section 124 of ultrasonic pulse transmitting/receiving device on the wall surface of container receiving section 121, and it measures temperature of the set metal container, that is, the temperature of liquid contained in the metal container. For the sensor unit 125 of temperature measuring device as such, an IR sensor, for example, may be used.

As the IR sensor, a radiation temperature sensor module IC of infrared sensor, which is a radiation temperature sensor, is preferred. It measures temperature based on measured infrared light and performs correction in consideration of radiation ratio, whereby accurate temperature can be measured in a moment.

Different from the head section 124 of ultrasonic pulse transmitting/receiving device and the sensor unit 125 of temperature measuring device, the pressing member is provided on stage 122. It sets a metal container using holder 123 and presses the metal container to head section 124 of ultrasonic pulse transmitting/receiving device.

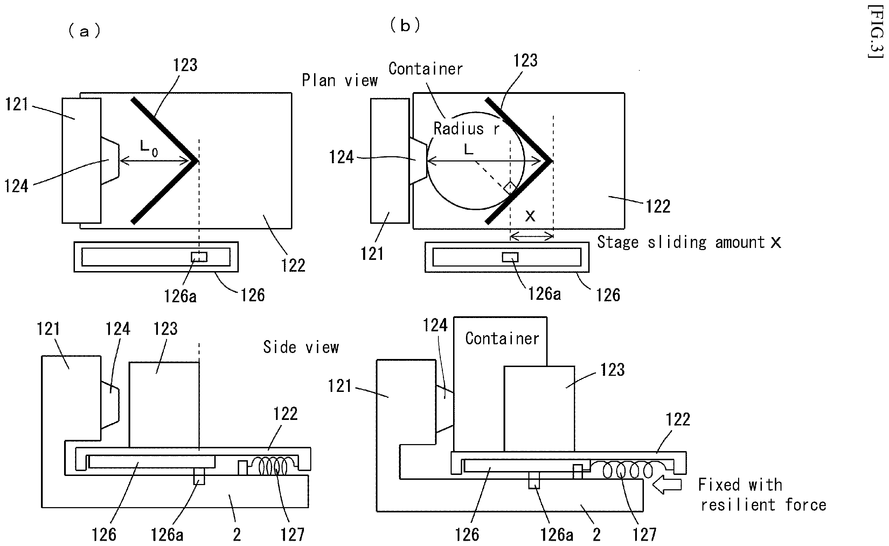

Fig. 3 includes schematic illustrations showing the pressing member of ultrasonic examination device, in which (a) is before setting the metal container and (b) is after setting the metal container. As shown in Fig. 3, the pressing member includes stage 122 slidable toward/away from container receiving section 121, and holder 123 provided on stage 122.

Here, below the lower surface of stage 122, a spring 127 is mounted in a compressed state, so that it exerts resilient force in the sliding direction of stage 122 as shown in Fig. 3(a).

When a metal container is set on holder 123, as shown in Fig. 3(b), stage 122 is slid to widen a distance $L_0$ between the head section 124 of ultrasonic pulse transmitting/receiving device and holder 123 to L. Spring 127 expands accordingly.

After the metal container is set, spring 127 is again compressed and, thus, the metal container is pressed against and fixed on the head section 124 of ultrasonic pulse transmitting/receiving device. Thus, examination can be conducted without any space generated between the metal container and the head section 124 of ultrasonic pulse transmitting/receiving device or between the metal container and holder 123. By detecting fixation of the metal container at the head section 124 of ultrasonic pulse transmitting/receiving device, examination can be started automatically.

Further, below the lower surface of stage 122, a variable resistor 126 sliding with stage 122 is mounted, and a sliding terminal 126a is fixed on the body 2 of examination device.

Therefore, when variable resistor 126 slides with stage 122, the distance from one end of variable resistor 126 to the sliding terminal 126a changes linearly, and accordingly, electric resistance of variable resistor 126 changes linearly in accordance with the distance X of sliding. Therefore, by checking the change in electric resistance, the propagation path length can be measured. Though variable resistor 126 is shown beside stage 122 in Fig. 3 for easier understanding, actually, it is mounted on the upper portion of the body 2 of examination device, below stage 122.

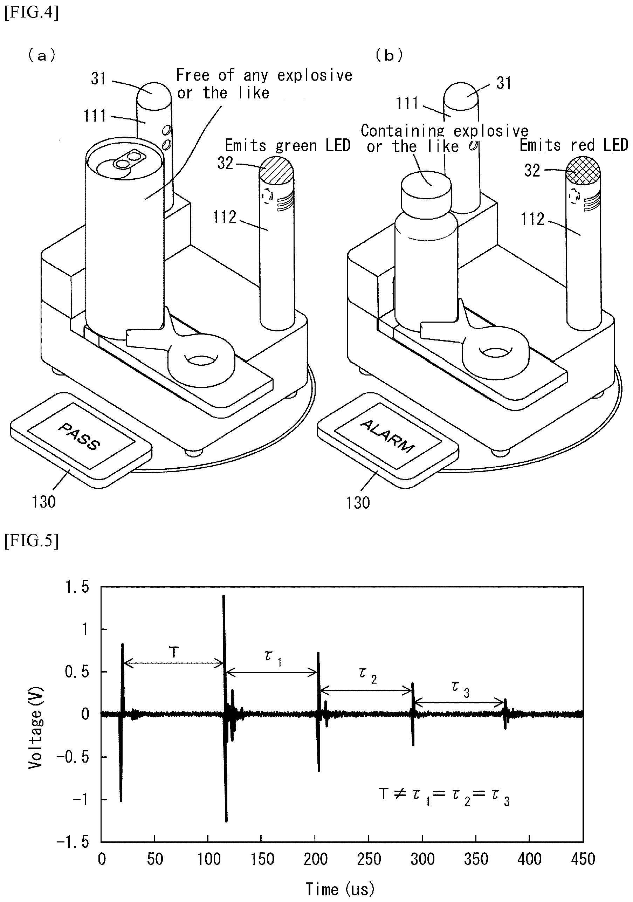

Fig. 4 includes perspective views showing the results of examination carried out by the ultrasonic examination device. When the liquid in a metal container is free of any explosive or the like, LED 32 and/or LED 31 emits green light as shown in Fig. 4(a), notifying the examiner that the liquid does not contain any explosive or the like and it is safe. On the other hand, if the liquid in the metal container is an explosive or the like, LED 32 and/or LED 31 emits red light as shown in Fig. 4(b), warning the examiner that the liquid possibly contains an explosive or the like. In Fig. 4, in addition to indication by LED, display unit 130 is separately provided, on which the result of examination is given by texts, "PASS" and "ALARM."

(2) Ultrasonic examination method

Next, a liquid examination method by the above ultrasonic examination device (ultrasonic examination method) will be explained.

Prior to examination, ultrasonic velocities in liquids containing explosives and the like and generally used liquids such as beverages are measured in advance and the results are stored as database in the ultrasonic examination device.

Since ultrasonic velocity in liquid is susceptible to the influence of liquid temperature, it is preferred to store ultrasonic velocities in the database additionally taking into consideration the temperature dependency.

Thereafter, a metal container is set and the ultrasonic velocity is measured. At this time, the temperature of liquid in the metal container is also measured, by the sensor unit 125 (see Fig. 1) provided above the head section 124 of ultrasonic pulse transmitting/receiving device.

Next, based on the measured ultrasonic velocity and the temperature, comparison with various ultrasonic velocities recorded in the database is conducted. From the results of comparison, data having the highest similarity is extracted and thereby the extracted liquid is determined to be the liquid as the object of examination, and whether it is a dangerous substance or safe substance is determined.

(3) Specific Example

In the following, a specific example of examination conducted using the above-described examination device will be described.

First, as shown in Fig. 3, a metal container having a radius r (diameter D = 2r) is set on holder 123 and pressed to head section 124 of the ultrasonic pulse transmitting/receiving device. Thus, ultrasonic signals are transmitted/received by the head section 124 of ultrasonic pulse transmitting/receiving device, a distance L between the head section 124 of ultrasonic pulse transmitting/receiving device and holder 123 is calculated based on the sliding distance X of variable resistor 126, and based on this value L, propagation path length (double the diameter D of metal container) 2D is calculated in accordance with the equation below.

[Formula 1]

$$2D = \frac{4L}{(1+\sqrt{2})}$$

Next, based on the received echo signals (reflected waves), propagation time in the solution is measured. As described above, the propagation time in a solution can be calculated based on time difference between the pulse reflected twice or more when the head section 124 of ultrasonic pulse transmitting/receiving device transmits an ultrasonic pulse having prescribed frequency and prescribed interval to the liquid in the metal container and generated echo signals (reflected waves) are received. Specific description will be given below.

Fig. 5 shows deviation between the first echo time T and each of echo intervals $\tau_1$ to $\tau_3$ measured by the ultrasonic pulse transmitting/receiving device. It can be seen from Fig. 5 that when the echo signals measured by the ultrasonic sensor are analyzed, there is a time difference between the time T from the start of pulse transmission (time: around 20 $\mu$s) to the first echo and each echo interval time $\tau_1$ to $\tau_3$ after the first echo.

Since $\tau_1$ to $\tau_3$ include propagation time inside the sensor, the propagation time from the piezoelectric element as the oscillation source of ultrasonic waves through the inside of the ultrasonic sensor must be checked. Therefore, identical sensors are placed opposite to each other and time necessary for signal transmission/reception was measured. It was found that it took 12 $\mu$s from the transmitting side to the receiving side.

The time of arrival of each echo emitted from the ultrasonic sensor to the container (echo arrival time) includes propagation time t' inside the ultrasonic sensor and, therefore, it is impossible to measure the time t of reciprocation in the solution based only on the time of arrival of the first echo. It is possible, however, to accurately measure the propagation time $\tau$ in the solution by calculating the interval between arrival time of each echo (for example, (second echo time) - (first echo time) = $\tau$).

When the interval between each of the echo arrival time is to be calculated, calculation can easily be performed by regarding the maximum value of each echo as the center of the echo. The position of the maximum value, however, is not always the same because of noise or the like, and hence, the obtained time may possibly be incorrect.

As a method of processing for eliminating such an error, it is preferable to use auto-correlation processing of echo waveforms, based on the nature of ultrasonic waves that the wave propagates in a solution at the same velocity for the same distance and basically the echo interval is equal and the waveform does not change.

Next, from the propagation path length (double the diameter D of metal container) 2D and on the propagation time $\tau$, the ultrasonic velocity v is calculated in accordance with the equation below.

[Formula 2]

$$v = 2D / \tau$$

Then, by comparing the calculated actual ultrasonic velocity value v and the ultrasonic velocities stored in the database, the ultrasonic velocity in the database closest to the actually measured ultrasonic velocity v is extracted, thereby the kind of liquid corresponding to the ultrasonic velocity is determined to be the liquid as the object of examination, and whether it contains any explosive or the like is determined.

As already described, ultrasonic velocities (sound velocities) in various liquids have temperature dependency and, therefore, it is necessary to take into consideration the temperature dependency in forming the database, and the same applies to the measured ultrasonic velocity.

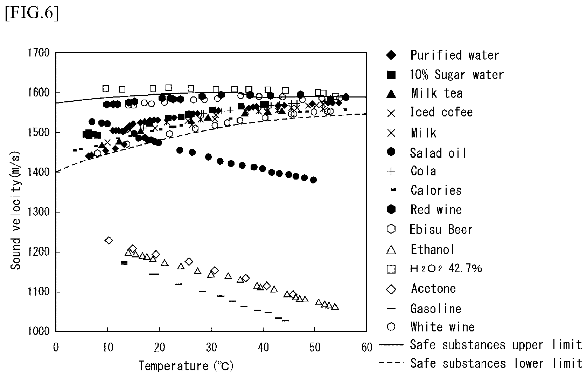

Fig. 6 shows measurement results of temperature dependency of sound velocity for various kinds of liquid. In Fig. 6, the ordinate represents sound velocity and the abscissa represents temperature.

It can be seen from Fig. 6 that sound velocities of safe substances such as purified water, 10% sugar water and milk tea fall within a region between two curves (solid line and dotted line), while the sound velocities of dangerous substances such as 42.7% hydrogen peroxide solution and acetone are out of the region defined by the two curves.

Accordingly, it is understood that in Fig. 6, the region between the two curves (solid line and dotted line) is the safe region, and whether the object of examination is safe or not can be determined depending on whether its result of measurement falls within this region or outside this region.

After the end of determination, the container set between the head section 124 of ultrasonic pulse transmitting/receiving device and the holder 123 is removed and the stage 122 is returned to the initial position.

4. Conclusion

As described above, according to the present embodiment, one liquid examination device is formed by integrating a near-infrared light examination device and an ultrasonic examination device, and examination can be done by switching the examination device to be applied depending on whether a container is optically transparent or not. Therefore, it is possible by one device to promptly and reliably examine, from outside of a container, whether any explosive or the like is contained in a liquid filled in the container, independent of the size or whether the container is optically transparent or not, or regardless of the remaining amount of liquid in the container or the label position.

The present invention has been described above with reference to the embodiments. However, the present invention is not limited to said embodiments. Various changes may be made on said embodiments within the scope identical or equivalent to that of the present invention.

[DESCRIPTION OF THE REFERENCE NUMERALS]

| | |
|---|---|
| 1 | Liquid examination device |
| 2 | Body of the examination device |
| 11 | Near-infrared light examination device |
| 12 | Ultrasonic examination device |
| 21 | Upper base |
| 31, 32 | LED |
| 111 | First pole |
| 112 | Second pole |
| 113 | First near-infrared light irradiating means |
| 114 | Second near-infrared light irradiating means |
| 115 | Near-infrared light receiving means |
| 121 | Container receiving section |
| 122 | Stage |
| 123 | Holder |
| 124 | Head section of ultrasonic pulse transmitting/receiving device |
| 125 | Sensor unit of temperature measuring device |
| 126 | Variable resistor |
| 126a | Sliding terminal |
| 127 | Spring |
| 130 | Display unit |

**Claims**

1. A liquid examination device for checking whether an explosive, a raw-material for explosive and/or an illicit drug is contained in a liquid filled in a container, comprising, as integrally formed components:

   a near-infrared light examination device irradiating said liquid with near-infrared light from outside an optically transparent container, receiving said near-infrared light transmitted through the liquid or said near-infrared light scattered by the liquid, and analyzing absorption spectrum of the received light, and thereby examining whether an explosive, a raw material for explosive and/or an illicit drag is contained in the liquid filled in said optically transparent container; and an ultrasonic examination device receiving reflected wave of ultrasonic wave applied to said liquid from outside a metal container, analyzing the ultrasonic velocity and thereby examining whether an explosive, a raw material for explosive and/or an illicit drag is contained in the liquid filled in said metal container.

2. The liquid examination device according to claim 1 wherein analysis of absorption spectrum of said near-infrared light by said near-infrared light examination device is conducted by comparing with database formed in advance on relations between explosives, raw materials for explosives and/or illicit drugs and their absorption spectra of near infrared light.

3. The liquid examination device according to claim 1 or 2, wherein said near-infrared light examination device includes two poles erected approximately vertically and spaced by a prescribed distance from each other, and a liquid in an optically transparent container placed between said two poles is irradiated with near-infrared light.

4. The liquid examination device according to claim 3 wherein
   an irradiating means for irradiating a liquid contained in an optically transparent container placed between said two poles with near-infrared light is provided on each of said two poles, and
   a light receiving means for receiving near-infrared light transmitted through the liquid or near-infrared light scattered by the liquid is provided on either one of said two poles.

5. The liquid examination device according to claim 4 wherein
   said light receiving means includes a light receiving mirror, and said received near-infrared light is reflected by said light receiving mirror before the absorption spectrum of said near-infrared light is measured.

6. The liquid examination device according to claim 4 or 5, wherein
   irradiation of near-infrared light by said irradiating means and reception of near-infrared light by said light receiving means are conducted constantly.

7. The liquid examination device according to claim 6 wherein
   said near-infrared light examination device includes a reference data updating function of updating reference data based on a light source spectrum of said near-infrared light received immediately before.

8. The liquid examination device according to claim 6 or 7, wherein
   placement of said optically transparent container between said two poles is detected by a reduction of said received near-infrared light, and examination by said near-infrared light examination device is automatically started.

9. The liquid examination device according to any one of claims 1 to 8, wherein
the device has, as database, absorption spectra of liquids known in advance as explosives, raw-materials for explosives and/or illicit drugs and of liquids known in advance as safe substances,
correlation coefficient of absorption spectrum of a measured liquid and each of the absorption spectra recorded in said database is calculated, and
by selecting the closest spectrum from said database based on said calculated correlation coefficient, examination is conducted as to whether the measured liquid is an explosive, a raw-material for an explosive and/or an illicit drug, or a safe substance.

10. The liquid examination device according to any one of claims 1 to 9, wherein
analysis of the ultrasonic velocity by said ultrasonic examination device is conducted by comparing with database formed in advance on relations between explosives, raw materials for explosives and/or illicit drugs and their ultrasonic velocities.

11. The liquid examination device according to claim 10, comprising
an ultrasonic pulse transmitting/receiving device transmitting an ultrasonic pulse to said liquid from outside of a metal container and applying the ultrasonic pulse to said liquid, and receiving reflected wave of said ultrasonic wave,
a propagation path length measuring device measuring propagation path length based on a diameter of said metal container,
a propagation time measuring device measuring propagation time based on time interval of said reflected wave received when said ultrasonic pulse is reflected a number of times, and
a temperature measuring device measuring temperature of said metal container.

12. The liquid examination device according to claim 11, wherein
said ultrasonic examination device may include a container receiving surface on which said metal container is set, and a pressing member pressing said metal container set on said container receiving surface to a head section transmitting/receiving an ultrasonic signal of said ultrasonic pulse transmitting/receiving device, and
said propagation path length measuring device may calculate the propagation path length in said metal container based on the position of said pressing member.

13. The liquid examination device according to claim 11 or 12, wherein
said ultrasonic examination device may examine whether or not said liquid contains an explosive, a raw-material for an explosive or an illicit drug,

based on said measured propagation path length and the ultrasonic velocity in said liquid calculated from said propagation path length and said propagation time, and on the temperature of said liquid measured separately.

14. The liquid examination device according to claim 12 or 13, wherein
the examination by said ultrasonic examination device may be automatically started upon detection of said metal container pressed against the head section transmitting/receiving an ultrasonic signal of said ultrasonic pulse transmitting/receiving device.

15. The liquid examination device according to any one of claims 1 to 14, wherein
an alarm means for notifying the result of examination by light and/or sound is provided, further.

16. The liquid examination method for examining whether an explosive, a raw-material for explosive and/or an illicit drug is contained in a liquid filled in a container using the liquid examination device according to any one of claims 1 to 15.

[FIG.1]

[FIG.2]

(a)
Free of any explosive
or the like

(b)
Containing explosive
or the like

31

111

Emits green LED

32

112

130

31

111

Emits red LED

32

112

130

[FIG.3]

[FIG.4]

(a)

31

111

Free of any explosive
or the like

Emits green LED

32

112

PASS

130

(b)

31

111

Containing explosive
or the like

Emits red LED

32

112

ALARM

130

[FIG.5]

$T \neq \tau_1 = \tau_2 = \tau_3$

[FIG.6]

## INTERNATIONAL SEARCH REPORT

| International application No. |
|---|
| PCT/JP2015/078472 |

A.  CLASSIFICATION OF SUBJECT MATTER
*G01N21/359*(2014.01)i, *G01N29/024*(2006.01)i

According to International Patent Classification (IPC) or to both national classification and IPC

B.  FIELDS SEARCHED

Minimum documentation searched (classification system followed by classification symbols)
G01N21/00-G01N21/01, G01N21/17-G01N21/61, G01N29/00-G01N29/52

Documentation searched other than minimum documentation to the extent that such documents are included in the fields searched
Jitsuyo Shinan Koho        1922-1996   Jitsuyo Shinan Toroku Koho   1996-2015
Kokai Jitsuyo Shinan Koho  1971-2015   Toroku Jitsuyo Shinan Koho   1994-2015

Electronic data base consulted during the international search (name of data base and, where practicable, search terms used)
JSTPlus(JDreamIII)

C.  DOCUMENTS CONSIDERED TO BE RELEVANT

| Category* | Citation of document, with indication, where appropriate, of the relevant passages | Relevant to claim No. |
|---|---|---|
| Y | JP 2011-220710 A  (Osaka University), 04 November 2011 (04.11.2011), claim 1 (Family: none) | 1-16 |
| Y | JP 2005-536726 A  (Battelle Memorial Institute), 02 December 2005 (02.12.2005), claims 1, 48; paragraphs [0003], [0007], [0180] & US 2004/0035208 A1 claims 1, 48; paragraphs [0003], [0007], [0216] & WO 2004/019028 A2    & EP 1540328 A | 1-16 |

☐  Further documents are listed in the continuation of Box C.      ☐  See patent family annex.

| * | Special categories of cited documents: |
|---|---|
| "A" | document defining the general state of the art which is not considered    to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 11 December 2015 (11.12.15) | 22 December 2015 (22.12.15) |

| Name and mailing address of the ISA/ | Authorized officer |
|---|---|
| Japan Patent Office 3-4-3,Kasumigaseki,Chiyoda-ku, Tokyo 100-8915,Japan | |
| | Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5207462 B

- JP 2011220710 A